(19) Europäisches Patentamt
European Patent Office
Office européen des brevets

(11) Numéro de publication: **0 284 781 B1**

(12) **FASCICULE DE BREVET EUROPEEN**

(45) Date de publication de fascicule du brevet: **16.10.91**

(51) Int. Cl.$^5$: **C07D 241/44**

(21) Numéro de dépôt: **88102840.1**

(22) Date de dépôt: **25.02.88**

# (54) Procédé pour la fabrication d'une 2,3-dihydroxyquinoxaline.

(30) Priorité: **06.03.87 FR 8703227**

(43) Date de publication de la demande: **05.10.88 Bulletin 88/40**

(45) Mention de la délivrance du brevet: **16.10.91 Bulletin 91/42**

(84) Etats contractants désignés: **AT BE CH DE ES GB IT LI NL SE**

(56) Documents cités:

**JOURNAL OF THE CHEMICAL SOCIETY, 1948, pages 519-523, Londres, GB; G.T. NEWBOLD et al.: "The oxidation of 2-hydroxyquinoxaline and its derivatives with hydrogen peroxide"**

(73) Titulaire: **INTEROX Société Anonyme**
**Rue du Prince Albert, 33**
**B-1050 Bruxelles(BE)**

(72) Inventeur: **Deschrijver, Paul**
**Tuitenberg naar Lombeekstraat, 46**
**B-1680 Lennik(BE)**
Inventeur: **Ganhy, Jean-Pierre**
**rue des Bégonias, 81**
**B-1170 Bruxelles(BE)**

(74) Mandataire: **Lederer, Franz, Dr. et al**
**Lederer, Keller & Riederer Patentanwälte**
**Lucile-Grahn-Strasse 22**
**W-8000 München 80(DE)**

**EP 0 284 781 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé pour la fabrication d'une 2,3-dihydroxyquinoxaline selon lequel on oxyde une quinoxaline au moyen de peroxyde d'hydrogène dans un milieu solvant.

Les 2,3-dihydroxyquinoxalines sont connues depuis longtemps et trouvent de nombreuses applications en photographie, comme insecticides et comme additifs de polymérisation pour améliorer les propriétés des polymères.

On sait depuis longtemps que la 2,3-dihydroxyquinoxaline peut être obtenue par réaction d'o-phéylènediamine avec l'acide oxalique ou avec un ester de l'acide oxalique en milieu acide chlorhydrique (BEILSTEIN Band XXIV, Syst. No. 3591, pages 380-381, *page 380*). Par ailleurs, on a proposé diverses méthodes de synthèse de la 2,3-dihydroxyquinoxaline par oxydation de quinoxalines substituées au moyen de peroxyde d'hydrogène en milieu acide acétique (JOURNAL OF CHEMICAL SOCIETY, 1948, London, G.T. NEWBOLD et F.S. SPRING "The oxidation of 2-hydroxyquinoxaline and its Derivatives with Hydrogen Peroxide", pages 519 à 522, *pages 522 et 523*; Ibid., 1956, London , J.K. LANDQUIST et J.A. SILK "Quinoxaline N-oxides, Part IV. Derivatives of Py-Hydroxylalkyl-, Aminoalkyl-, and -Carboxy-quinoxalines", pages 2052 à 2058, *page 2058*; Ibid., 1957, London, G.W.H. CHEESEMAN "Quinoxalines and Related Compounds. Part III. Some 2-Substituted Quinoxalines", pages 3236 à 3239, *page 3238*).

Ces procédés connus ne permettent cependant pas d'obtenir les dérivés substitués de la 2,3-dihydroxyquinoxaline avec de bons rendements. Ils présentent de plus le désavantage de nécessiter des durées de réaction très longues.

L'invention remédie à ces inconvénients des procédés connus en fournissant un procédé nouveau de fabrication de 2,3-dihydroxyquinoxalines substituées ou non substituées dont les rendements sont plus élevés que ceux des procédés connus, avec des durées de réaction sensiblement plus courtes.

A cet effet, l'invention concerne un procédé pour la fabrication d'une 2,3-dihydroxyquinoxaline par oxydation d'une quinoxaline au moyen de peroxyde d'hydrogène dans un solvant inerte, selon lequel on exécute l'oxydation en présence d'eau et d'un catalyseur sélectionné parmi le sélénium et les composés du sélénium.

On entend désigner par l'expression 2,3-dihydroxyquinoxalines, un composé de formule générale :

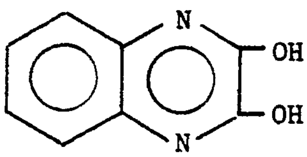

dans laquelle les atomes d'hydrogène du noyau benzénique peuvent être non substitués ou substitués.

La quinoxaline mise en oeuvre peut être de la quinoxaline non substituée ou une quinoxaline substituée sur le noyau benzénique. En variante, on peut mettre en oeuvre un mélange de plusieurs quinoxalines substituées ou on mélange de quinoxaline non substituée avec une ou plusieurs quinoxalines substituées.

Des exemples de substituants qui conviennent sont ceux qui constituent des groupes électrodonneurs tels que les groupes méthyle, éthyle, n-propyle ou isopropyle.

Les substituants qui constituent des groupes électrocapteurs conviennent aussi. Des exemples de tels substituants sont les groupes halogène, amine ou nitro. Ou peut aussi utiliser des quinoxalines substituées par plus d'un substituant. De telles quinoxalines polysubstituées peuvent comporter un ou plusieurs substituants électrodonneurs et un ou plusieurs substituants électrocapteurs sur le noyau benzénique.

Selon l'invention, le catalyseur mis en oeuvre peut consister en sélénium métal, en oxyde de sélénium, en acide sélénieux ou en acide sélénique. On peut aussi utiliser des composés organiques du sélénium, notamment des composés organoséléniés. On a constaté que l'emploi de l'oxyde de sélénium est particulièrement avantageux.

Le catalyseur est le plus souvent mis en oeuvre en une concentration comprise entre 9 et 150 mmoles par mole de quinoxaline. Pour éviter la formation d'une trop grande quantité de monooxyquinoxaline, il est préférable de mettre en oeuvre plus de 25 mmoles de catalyseur par mole de quinoxaline. De même, en vue de limiter la formation de dioxyquinoxaline, on préfère ne pas dépasser une teneur de 80 mmoles de catalyseur par mole de quinoxaline. Le catalyseur peut être incorporé au milieu réactionnel à l'état pour, en solution ou en dispersion dans un solvant.

Le peroxyde d'hydrogène utilisé pour oxyder les quinoxalines selon le procédé de l'invention peut être mis en oeuvre à l'état anhydre, sous la forme d'une solution aqueuse commerciale ou sous la forme d'une solution dans un solvant organique. Les solutions aqueuses sont généralement préférées en raison de leur

moindre coût et de leur plus grande disponibilité. Des solutions aqueuses contenant au moins 10 % en poids et pas plus de 95 % en poids de peroxyde d'hydrogène conviennent bien. De préférence, on emploie des solutions qui contiennent entre 20 et 90 % en poids de peroxyde d'hydrogène.

Le solvant inerte du procédé selon l'invention a pour fonction de réaliser un milieu liquide homogène capable, dans les conditions de la réaction, de solubiliser la quinoxaline, le peroxyde d'hydrogène et la 2,3-dihydroxyquinoxaline formée. Il doit être inerte vis-à-vis de la quinoxaline mise en oeuvre, du peroxyde d'hydrogène et des produits de l'oxydation. Il peut être un solvant organique résistant à l'oxydation par le peroxyde d'hydrogène dans les conditions de la réaction ou un mélange d'un ou plusieurs de ces solvants avec une quantité d'eau telle que la proportion pondérale eau/solvant soit comprise entre 0 et 0,4. Les solvants organiques les plus avantageux sont ceux sélectionnés parmi les alcools aliphatiques, le cyclohexanol, l'alcool benzylique et le dioxanne. Conviennent particulièrement bien, l'éthanol, le n-propanol, le n-butanol et le polyéthylèneglycol.

La quantité de solvant inerte à utiliser n'est pas critique. Elle dépend de divers facteurs tels que la nature de la quinoxaline, la concentration de la solution de peroxyde d'hydrogène, la solubilité de la 2,3-dihydroxyquinoxaline dans le milieu réactionnel et la technique pour l'extraire hors de ce milieu. Elle peut se déterminer dans chaque cas d'espèce au moyen d'un nombre limité d'essais de laboratoire. Généralement, la quantité pondérale de solvant est comprise entre 2 et 50 fois le poids de quinoxaline mise en oeuvre. Le plus souvent, ce poids de solvant est supérieur à 4 fois le poids de quinoxaline et ne dépasse pas 20 fois ce poids. Un poids de solvant compris entre 8 et 12 fois le poids de quinoxaline mise en oeuvre convient particulièrement bien.

Selon l'invention, l'oxydation de la quinoxaline par le peroxyde d'hydrogène doit être opérée en présence d'eau. Celle-ci peut être constituée essentiellement de l'eau produite lors de la réaction. En variante, elle peut comprendre un surplus d'eau par rapport à celle provenant de la réaction d'oxydation; ce surplus d'eau peut par exemple accompagner le solvant inerte et/ou le peroxyde d'hydrogène lorsque celui-ci est mis en oeuvre à l'état d'une solution aqueuse. En règle générale, il s'est avéré souhaitable de mettre en oeuvre un surplus pondéral d'eau au moins égal à 1 % du poids du solvant inerte, mais n'excédant pas, de préférence, 50 % de ce poids. Le surplus pondéraux d'eau compris entre 5 et 35 % du poids de solvant inerte sont préférés.

La température et la pression auxquelles on effectue la réaction d'oxydation selon l'invention ne sont pas critiques et peuvent varier dans de larges limites. Elles dépendent de la nature de la quinoxaline à oxyder et de celle du solvant inerte. Elles conditionnent aussi la durée de la réaction et sont à déterminer dans chaque cas particulier au moyen d'essais de laboratoire de routine.

La durée de la réaction dépend de la nature de la quinoxaline à oxyder ainsi que du catalyseur et du solvant mis en oeuvre. Elle peut varier entre 2 et 25 heures, en fonction de la température.

Après réaction, le mélange peut être soumis à diverses techniques de séparation telles que la distillation, la décantation et la filtration pour recueillir la 2,3-dihydroxyquinoxaline formée et les réactifs non transformés. Dans une forme de réalisation particulière de l'invention, on récupère les réactifs non transformés à l'issue de l'opération de séparation. Ils peuvent alors être avantageusement recyclés dans le procédé.

Le procédé suivant l'invention peut être mis en oeuvre en continu ou en discontinu, dans un réacteur unique ou dans une série de réacteurs en parallèle ou en série de types usuels convenant pour les mélanges réactionnels liquides.

Le catalyseur et les réactifs peuvent être introduits de diverses manières connues en soi. On peut ainsi procéder, selon les cas d'espèce, à une introduction étagée du catalyseur, de la quinoxaline et/ou du peroxyde d'hydrogène ou à un mélange simultané de ces constituants.

Le procédé selon l'invention présente l'avantage de fournir des 2,3-dihydroxyquinoxalines substituées ou non substituées avec de meilleurs rendements que ceux obtenus avec les procédés connus.

Afin d'illustrer l'invention, sans pour autant en limiter la portée, on donne ci-après des exemples de fabrication de 2,3-dihydroxyquinoxalines (exemples 1 à 4 et exemple 7) au moyen du procédé selon l'invention. Les exemples 5R, 6R et 8R concernent des essais de référence, non conformes à l'invention.

Exemples 1 à 6R

Dans un réacteur à double enveloppe muni d'un système de réfrigération interne, d'un agitateur magnétique et d'un dispositif d'introduction de réactif liquide, on a introduit 200 mg (1,8 mmoles) de $SeO_2$, 50 ml de solvant et 6,5 g (50 mmoles) de quinoxaline.

On a ensuite amené la température du mélange à 70° C puis on a introduit 6 ml d'une solution de $H_2O_2$ à 85 % en poids (200 mmoles) en 1 minute via le dispositif d'introduction de réactif liquide. Après plusieurs

minutes, un précipité brun s'est formé. Après avoir laissé réagir pendant une durée suffisante, on a arrêté la réaction par refroidissement du milieu réactionnel.

Les produits de la réaction ont ensuite été analysés dans le précipité (par résonnance magnétique nucléaire et chromatographie en couche mince) et dans le filtrat (par chromatographie en couche mince).

Les essais 1 à 4 ont été effectués conformément à l'invention en n'éliminant pas l'eau du milieu réactionnel. Dans l'essai 1, le solvant a consisté en n-propanol anhydre. Dans les essais 2,3 et 4, on a utilisé en guise de solvant inerte un mélange de n-propanol et d'eau dans le but d'accroître volontairement la teneur en eau du milieu réactionnel.

L'essai 5R a été effectué à titre de comparaison en éliminant en continu l'eau hors du milieu réactionnel grâce à l'utilisation d'un décanteur florentin en pied du réfrigérant.

L'essai 6R a été effectué sans catalyseur, à titre de référence.

Les résultats des analyses ainsi que la durée de la réaction et la composition du solvant sont donnés au tableau I qui suit.

Les mentions DHQ, MOQ et DOQ signifient, respectivement, 2,3-dihydroxyquinoxalines, monooxyquinoxalines et dioxyquinoxalines.

La dernière colonne du tableau I indique si l'analyse a été effectuée dans le filtrat (référence F) ou dans le précipité (référence P). En regard de la référence T, le tableau I donne aussi la somme totale des résultats obtenus dans le filtrat et dans le précipité.

Tableau I

| Exemple No. | Durée de la réaction, h | Rapport pondéral solvant/eau | Rendement molaire, % DHQ | MOQ | DOQ | |
|---|---|---|---|---|---|---|
| 1 | 17 | 100:0 | 5,0<br>19,5<br>24,5 | 17,8<br>0,2<br>35,8 | 13,6<br>0,1<br>27,4 | F<br>P<br>T |
| 2 | 17 | 95:5 | 7,4<br>22,0<br>29,4 | 38,4<br>0,4<br>38,8 | 19,8<br>0,2<br>20,0 | F<br>P<br>T |
| 3 | 17 | 90:10 | 7,4<br>22,6<br>30,0 | 41,0<br>0,8<br>41,8 | 9,8<br>0,2<br>10,2 | F<br>P<br>T |
| 4 | 17 | 75:25 | 9,8<br>15,2<br>25,0 | 41,0<br>0,0<br>41,0 | 9,8<br>0,0<br>9,8 | F<br>P<br>T |
| 5R | 2 | 100:0 | 4,2<br>4,4<br>8,6 | 30,2<br>4,0<br>34,2 | 6,6<br>18,8<br>25,4 | F<br>P<br>T |
| 6R | 17 | 100:0 | 0,2<br>0,0<br>0,2 | 16,8<br>0,0<br>16,8 | 0,0<br>0,0<br>0,0 | F<br>P<br>T |

La comparaison des résultats de l'exemple 1 avec ceux des exemples 5R et 6R montre l'avantage du procédé selon l'invention en ce qui concerne le rendement molaire en 2,3-dihydroxyquinoxaline obtenu.

Les exemples 2 à 4 mettent en évidence l'intérêt d'introduire une quantité supplémentaire d'eau avec les matières premières dans le milieu réactionnel.

Exemples 7 et 8R

Les exemples 7 et 8R ont été effectués dans les mêmes conditions que les exemples 1 à 5R excepté la nature du solvant inerte qui a été remplacé par du n-butanol.

Les résultats ont été portés au tableau II qui suit.

Tableau II

| Exemple No. | Durée de la réaction, h | Rapport pondéral solvant/eau | Rendement molaire, % | | | |
|---|---|---|---|---|---|---|
| | | | DHQ | MOQ | DOQ | |
| 7 | 17 | 100:0 | 4,4 | 20,5 | 7,4 | F |
| | | | 6,4 | 1,8 | 45,6 | P |
| | | | 10,8 | 22,3 | 53,0 | T |
| 8R | 17 | 100:0 | 2,2 | 10,3 | 3,7 | F |
| | | | 3,2 | 0,9 | 22,8 | P |
| | | | 5,4 | 11,2 | 26,5 | T |

## Revendications

1. Procédé de fabrication d'une 2,3 dihydroxyquinoxaline par oxydation d'une quinoxaline au moyen de peroxyde d'hydrogène dans un solvant inerte, caractérisé en ce qu'on exécute l'oxydation en présence d'eau et d'un catalyseur sélectionné parmi le sélénium et les composés du sélénium.

2. Procédé suivant la revendication 1, caractérisé en ce que la quinoxaline utilisée comme matière première est sélectionnée parmi la quinoxaline non substituée et les quinoxalines substituées sur le noyau benzénique.

3. Procédé suivant la revendication 1 ou 2, caractérisé en ce que le catalyseur mis en oeuvre consiste en sélénium métal, en oxyde de sélénium, en acide sélénieux, en acide sélénique ou en un composé organique du sélénium (composé organosélénié).

4. Procédé suivant la revendication 3, caractérisé en ce que le catalyseur mis en oeuvre consiste en oxyde de sélénium.

5. Procédé suivant une quelconques des revendications précédentes, caractérisé en ce que le catalyseur est utilisé en une quantité comprise entre 25 et 80 millimoles par mole de quinoxaline.

6. Procédé suivant une quelconque des revendications précédentes, caractérisé en ce que le solvant inerte est sélectionné parmi les alcools aliphatiques, le cyclohexanol, l'alcool benzylique et le dioxanne.

7. Procédé suivant la revendication 6, caractérisé en ce que l'alcool aliphatique est sélectionné parmi l'éthanol, le n-propanol, le n-butanol et le polyéthylèneglycol.

8. Procédé selon une quelconque des revendications précédentes, caractérisé en ce qu'une partie au moins de l'eau mise en oeuvre est de l'eau produits par la réaction d'oxydation.

9. Procédé suivant une quelconque des revendications précédentes, caractérisé en ce que l'on ajoute au milieu réactionnel une quantité d'eau au maximum égale à 35 % du poids de solvant inerte.

## Claims

1. Process for the production of a 2,3 dihydroxyquinoxaline by oxidation of a quinoxaline by means of hydrogen peroxyde in an inert solvent, characterized in that the oxidation is carried out in the presence of water and a catalyst selected from the group consisting of selenium and the selenium compounds.

2. Process according to claim 1, characterized in that the chinoxaline used as starting material is selected from the group consisting of the non-substituted chinoxaline and the chinoxalines substituted at the benzene nucleus.

3. Process according to claim 1 or 2, characterized in that the used catalyst consists of selenium metal, selenium oxide, selenious acid, selenic acid or an organic selenium compound (organoselenified compound).

4. Process according to claim 3 characterized in that the used catalyst consists of selenium oxide.

5. Process according to one of the preceding claims characterized in that the catalyst is used in a quantity between 25 and 80 millimoles per mol chinoxaline.

6. Process according to one of the preceding claims characterized in that the inert solvent is selected from the group consisting of the aliphatic alcohols, cyclohexanol, benzyl alcohol and dioxane.

7. Process according to claim 6 characterized in that the aliphatic alcohol is selected from the group consisting of ethanol, n-propanol, n-butanol and polyethyleneglycol.

8. Process according to one of the preceding claims characterized in that at least one part of the used water is water produced by the oxidation reaction.

9. Process according to one of the preceding claims characterized in that a water quantity of 35% at the most of the weight of the inert solvent is added to the reaction medium.

**Patentansprüche**

1. Verfahren zur Herstellung eines 2,3 Dihydroxychinoxalins durch Oxidation eines Chinoxalins mit Hilfe von Wasserstoffperoxyd in einem inerten Lösungsmittel, dadurch gekennzeichnet, daß man die Oxidation in Anwesenheit von Wasser und einem Katalysator, ausgewählt unter Selen und den Selenverbindungen durchführt.

2. Verfahren nach Anspruch 1, dadurch gekennzeichnet, daß das als Ausgangsmaterial verwendete Chinoxalin ausgewählt ist unter dem nicht-substituierten Chinoxalin und den auf dem Benzolkern substituierten Chinoxalinen

3. Verfahren nach Anspruch 1 oder 2, dadurch gekennzeichnet, daß der eingesetzte Katalysator aus Selenmetall, Selenoxid, seleniger Säure, Selensäure oder einer organischen Selenverbindung (Organoselenverbindung) besteht.

4. Verfahren nach Anspruch 3, dadurch gekennzeichnet, daß der eingesetzte Katalysator aus Selenoxid besteht.

5. Verfahren nach einem der vorhergehenden Ansprüche, dadurch gekennzeichnet, daß der Katalysator in einer Menge zwischen 25 und 80 Millimol pro Mol Chinoxalin verwendet wird.

6. Verfahren nach einem der vorhergehenden Ansprüche, dadurch gekennzeichnet, daß das inerte Lösungsmittel ausgewählt ist unter den aliphatischen Alkoholen, Cyclohexanol, Benzylalkohol und Dioxan.

7. Verfahren nach Anspruch 6, dadurch gekennzeichnet, das der aliphatische Alkohol ausgewählt ist unter Ethanol, n-Propanol, n-Butanol und Polyethylenglykol.

8. Verfahren nach einem der vorhergehenden Ansprüche, dadurch gekennzeichnet, daß wenigstens ein

Teil des eingesetzten Wassers durch die Oxidationsreaktion hergestelltes Wasser ist.

9. Verfahren nach einem der vorhergehenden Ansprüche, dadurch gekennzeichnet, daß man dem Reaktionsmedium eine Menge an Wasser von höchstens gleich 35% des Gewichts des inerten Lösungsmittels zufügt.